# FASCICULE DE BREVET EUROPEEN

(11) **EP 0 822 825 B1**
(45) Date de publication et mention de la délivrance du brevet: **30.06.2004**
(21) Numéro de dépôt: 96914261.1
(22) Date de dépôt: 29.04.1996
(51) Int. Cl.: A61K 35/78, A23L 1/221, A23L 2/52, A23L 1/03, A23C 9/13

(54) **EXTRAIT FLAVONOIDIQUE DE GINKGO BILOBA DEPOURVU DE TERPENES ET A FORTE TENEUR EN HETEROSIDES FLAVONOIDIQUES**
FLAVONOIDEN ENTHALTENDE EXTRACTEN VON GINGKO BILOBA OHNE TERPENEN UND MIT EINEM HÖHEREN GEHOLT AN FLAVONOIDISEHEN HETEROSIDEN
GINKGO BILOBA FLAVONOID EXTRACT WHICH IS TERPENE-FREE AND HAS A HIGH FLAVONOID HETEROSIDE CONTENT

(30) Priorité: 27.04.1995 GB 9508533
(43) Date de publication de la demande: 11.02.1998
(73) Titulaire: SOCIETE DE CONSEILS DE RECHERCHES ET D'APPLICATIONS SCIENTIFIQUES (S.C.R.A.S.), 75016 Paris (FR)
(72) Inventeur: O'REILLY, Joseph, County Cork (IE)
(74) Mandataire: Bourgouin, André
(86) Numéro de dépôt international: PCT/FR1996/000653
(87) Numéro de publication internationale: WO 1996/033728

(56) Documents cités:
- EP-A- 0 360 556
- EP-A- 0 431 536
- EP-A- 0 436 129
- EP-A- 0 543 051
- EP-A- 0 577 143
- FR-A- 2 007 352
- DATABASE WPI Section Ch, Week 9517 Derwent Publications Ltd., London, GB; Class B04, AN 95-126099 XP002011383 & JP 07 048 267 A (ASAHI BREWERIES) , 21 Février 1995
- DATABASE WPI Section Ch, Week 8742 Derwent Publications Ltd., London, GB; Class BO4, AN 87-294414 XP002011384 & JP 62 205 028 A (DAICEL) , 9 Septembre 1987
- DATABASE WPI Section Ch, Week 9602 Derwent Publications Ltd., London, GB; Class B03, AN 96-018388 XP002011385 & KR 9 402 796 B (SUN KYONG) , 2 Avril 1994
- FOOD TECHNOLOGY, vol. 49, no. 9, 1 Septembre 1995, pages 64/65, 68-70, 72, XP000530189 "DEVELOPMENTS IN BEVERAGE ADDITIVES"

## Description

L'invention concerne un extrait flavonoïdique de ginkgo biloba, et plus spécifiquement un extrait subtantiellemement dépourvu de terpènes et à forte teneur en hétérosides flavonoïdiques. Cet extrait peut avantageusement être utilisé en tant qu'agent aromatisant. L'invention concerne également une composition aromatisante contenant un tel extrait et l'utilisation de cet extrait en tant qu'ingrédient aromatisant.

Les applications des extraits de feuilles de ginkgo biloba dans les domaines médical et cosmétique, sont bien connues. L'extrait EGb 761 est peut-être le plus connu dans le domaine médical (L'extrait de ginkgo biloba (EGb 761), La Presse Médicale, 1986, vol. 31, numéro spécial, Editions Masson). Cet extrait comprend principalement deux familles de substances : les substances flavonoïdiques et terpéniques . De nouveaux extraits ont été définis qui, de façon inattendue, modifient les propriétés organoleptiques de certains aliments tels que les boissons, les produits laitiers, les produits sucrés.

Un des aspects de la présente invention a donc pour objet des extraits qui ne contienent pas, ou très peu, de terpènes (ginkgolides et bilobalides) qui possèdent une grande activité thérapeutique. Par ailleurs, il est apparu intéressant d'obtenir des extraits enrichis en substances flavonoïdiques : ce sont essentiellement des mono, di et tri glucosides de Kaempférol, de Quercétine et d'Isorhamnétine avec du glucose et du rhamnose.

L'invention a ainsi pour objet un extrait flavonoïdique dépourvu de terpènes et à forte teneur en hétérosides flavonoïdiques. Cela signifie que l'extrait est dépourvu de terpènes et contient de 28 à 35 % d'hétérosides flavonoïdiques et de préférence 28 à 32 %.

Plus particulièrement, l'invention a pour objet un extrait flavonoïdique comprenant au maximum 1 % de terpènes et de 28 à 35 % d'hétérosides flavonoïdiques. De façon préférentielle, l'extrait comprend au maximum 0,5 % de terpènes et de 28 à 32 % d'hétérosides flavonoïdiques.

Un extrait flavonoïdique à forte teneur en hétérosides flavonoïdiques peut être obtenu par extraction sous vide partiel, par un mélange acétone-eau. Après des étapes de délipidation, d'élimination des substances indésirables par différents solvants et par précipitation, la solution extractive est concentrée et l'extrait est séché sous vide.

Le procédé de préparation d'un extrait dépourvu de terpènes tel que défini ci-dessus comprend plusieurs étapes d'extraction d'extrait de feuilles de ginkgo biloba par des solvants. Une des étapes d'extraction est une étape de déterpénation et le solvant utilisé est un composé de formule RC(O)OR' dans laquelle R et R' représentent, indépendamment, un alkyl inférieur, seul ou mélangé avec un hydrocarbure aliphatique saturé contenant au moins 5 atomes de carbone. L'étape d'extraction peut être effectuée à n'importe quel stade du procédé. De préférence, le solvant utilisé lors de l'étape de deterpénation, comprend de 0 à 20 % d'hydrocarbure aliphatique saturé. Un tel procédé de déterpénation est décrit dans le brevet EP 436 129.

Des étapes d'extraction autres que l'étape de déterpénation, sont connues dans la littérature notamment dans les brevets EP431535, EP 431536, EP 360556 et EP324197.

Dans les définitions indiquées ci-dessus, l'expression alkyle inférieure représente de préférence un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle. Les solvants de formule RC(O)OR'dans laquelle les radicaux R et R' représentent méthyle, éthyle ou propyle, et notamment l'acétate d'éthyle, sont préférentiellement utilisés.

L'hydrocarbure aliphatique saturé peut être choisi parmi l'hexane, l'heptane, l'octane. De préférence, l'heptane est utilisé.

Le procédé de préparation d'un extrait enrichi en hétérosides flavonoïdiques tel que défini ci-dessus comprend quant à lui plusieurs étapes d'extraction d'extrait de feuilles de ginkgo biloba par des solvants. Une des étapes d'extraction est une étape d'enrichissement en hétérosides flavonoïdiques et le solvant utilisé, en quantité minimum, est un alcool, seul ou mélangé avec une cétone, de préférence l'acétone. L'étape d'extraction peut être effectuée à n'importe quel stade du procédé. De préférence, l'alcool utilisé est un alcool inférieur tel que le méthanol, l'éthanol, le propanol, le butanol, et de préférence le butanol. La quantité de solvant utilisée peut être de 3 à 12 parties, et de préférence dans la partie inférieure de cette fourchette.

Un exemple est décrit ci-après dans la partie expérimentale ; l'ordre des étapes d'extraction par le mélange acétate d'éthyl/heptane et le mélange acétone/butanol peut être inversé.

Un extrait selon l'invention modifie simultanément le profil olfactif et le profil gustatif du produit testé. De par ses propriétés organoleptiques, cet extrait peut être utilisé pour la préparation de compositions aromatiques de type varié. Il peut être utilisé seul ou en association avec d'autres ingrédients aromatisants couramment employés. Ainsi, l'utilisation d'un tel extrait flavonoïdique est envisagé dans les produits alimentaires. Par produits alimentaires, on peut citer les produits laitiers comme par exemple les yaourts, les boissons rafraîchissantes ou nutritives, et plus particulièrement les boissons rafraîchissantes sans alcool telles que les boissons orangées ou toniques à base de quinine, mais également les produits sucrés comme par exemple les chewing-gums.

La quantité utilisée dans une composition aromatisante ou un produit aromatisé, peut varier dans une gamme étendue de valeurs ; cette valeur dépend bien entendu du produit dans lequel l'arôme ou la composition aromatique est incorporée, mais également de la nature et de la quantité des autres constituants de la composition aromatique et de l'effet désiré.

L'extrait flavonoïdique selon l'invention est utilisé à des concentrations comprises entre 0,001 et 0,1 % en poids par rapport au poids total du produit aromatisé, de préférence entre 0,001 et 0,05 %. Dans le cas de produits laitiers, la concentration d'extrait flavonoïdique peut être comprise entre 0,005 et 0,045 %. Dans le cas de produits tels que les chewing-gums, la concentration d'extrait flavonoïdique peut être comprise entre 0,01 et 0,04 %. Dans le cas de produits alimentaires tels que les boissons rafraîchissantes ou nutritives, la concentration d'extrait flavonoïdique peut être comprise entre 0,002 et 0,02 %.

Les exemples suivants sont présentés pour illustrer l'invention.

### Exemple 1 : Procédé d'obtention d'un extrait flavonoïdique

Les feuilles de ginkgo biloba sont extraites avec de 6 à 12 parties (de préférence 8) d'eau contenant 60 % d'acétone à 50-60° C et la solution est concentrée de manière à réduire le pourcentage en acétone à moins de 3 %. Cette solution est refroidie et les lipides sont éliminés par décantation. La solution aqueuse est extraite avec de 2 à 5 parties d'acétate d'éthyl contenant de 0 à 20 % d'heptane. La solution résultante est extraite avec une quantité minimum d'un mélange acétone / butanol (pourcentage d'acétone 0 à 15 %) en présence de sulfate d'ammonium. La phase organique est concentrée ; après ajout d'éthanol, la solution est concentrée de nouveau. Après une nouvelle dilution à l'éthanol, la solution est refroidie et des précipités insolubles sont filtrés. La solution résultante est concentrée, séchée et enfin broyée pour récupérer l'extrait flavonoïdique sous forme d'une poudre homogène.

### Exemple 2 : Analyse sensorielle

Une analyse sensorielle d'un extrait flavonoïdique tel qu'obtenu dans l'exemple 1, a été menée pour un certain nombre de produits alimentaires (produits laitiers, boissons rafraîchissantes et chewing-gums), avec ou sans extrait flavonoïdique.

60 dégustateurs ont participé à cette analyse. Pour chaque item sensoriel (acide, amer, sucré, salé), chaque dégustateur indique une note d'intensité de 0 à 5, correspondant à un degré croissant d'appréciation du produit testé.

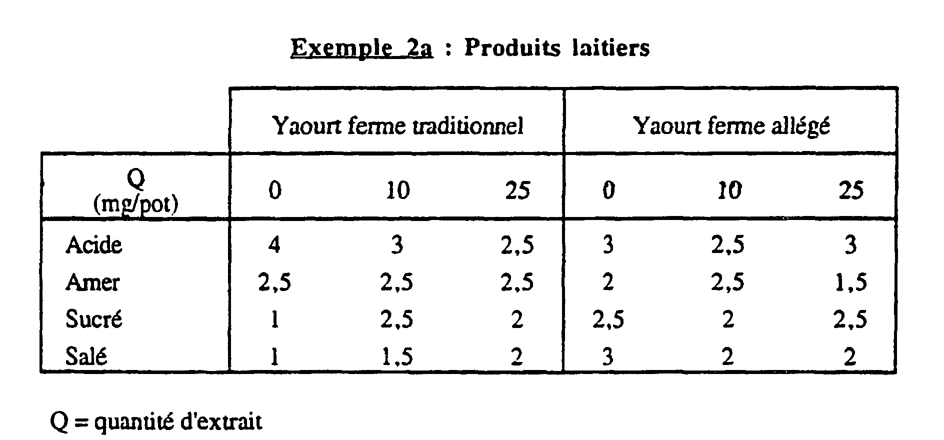

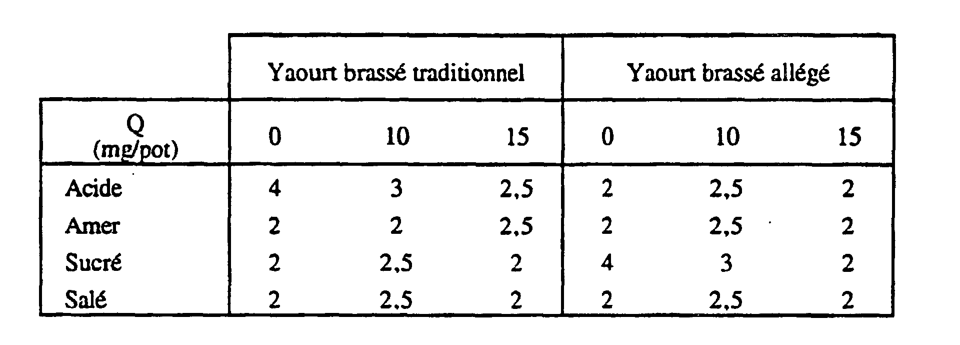

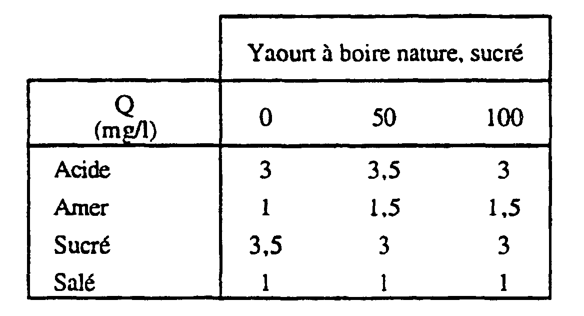

L'astringence naturelle de l'extrait équilibre bien les saveurs fondamentales des produits laitiers en atténuant l'acidité des produits traditionnels. L'extrait apparaît très complémentaire des notes laitières et fruitées.

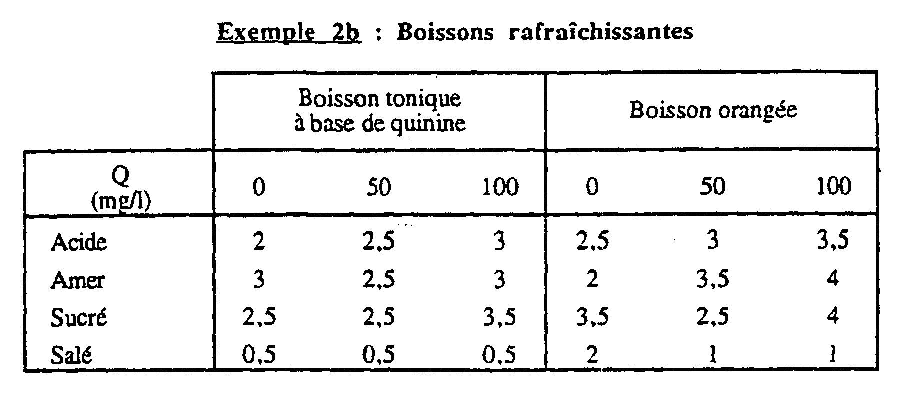

L'astringence de l'extrait se marie harmonieusement avec celle des boissons ; et plus particulièrement avec celle des boissons à note quinine et / ou écorces amères telles que l'Indian Tonic®.

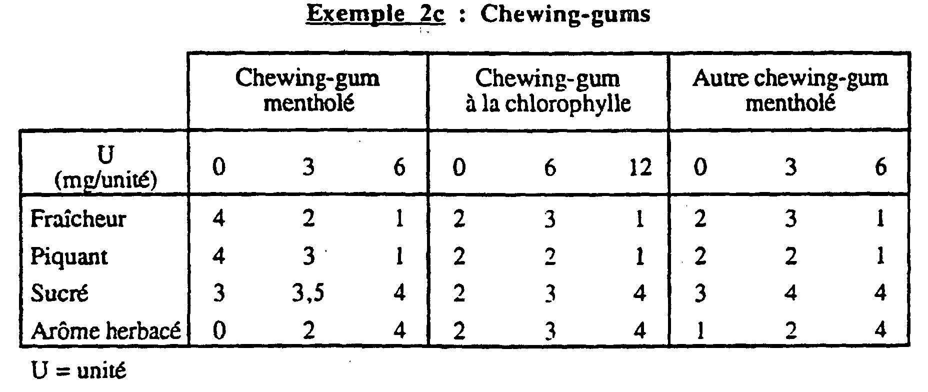

### Exemple 3 : Test descriptif

Ce test a été mené sur plusieurs variétés de yaourts. 60 dégustateurs, consommateurs de yaourts natures, ont participé au test. La méthode utilisée est un test comparatif de profils sensoriels. Le questionnaire comporte 9 ou 10 items descriptifs suivant la variété testée "ferme" ou "brassé". Item après item, chaque dégustateur indique sur une échelle non structurée de 10 cm, l'intensité perçue et ceci successivement pour la totalité des produits. Les résultats obtenus sont les suivants :

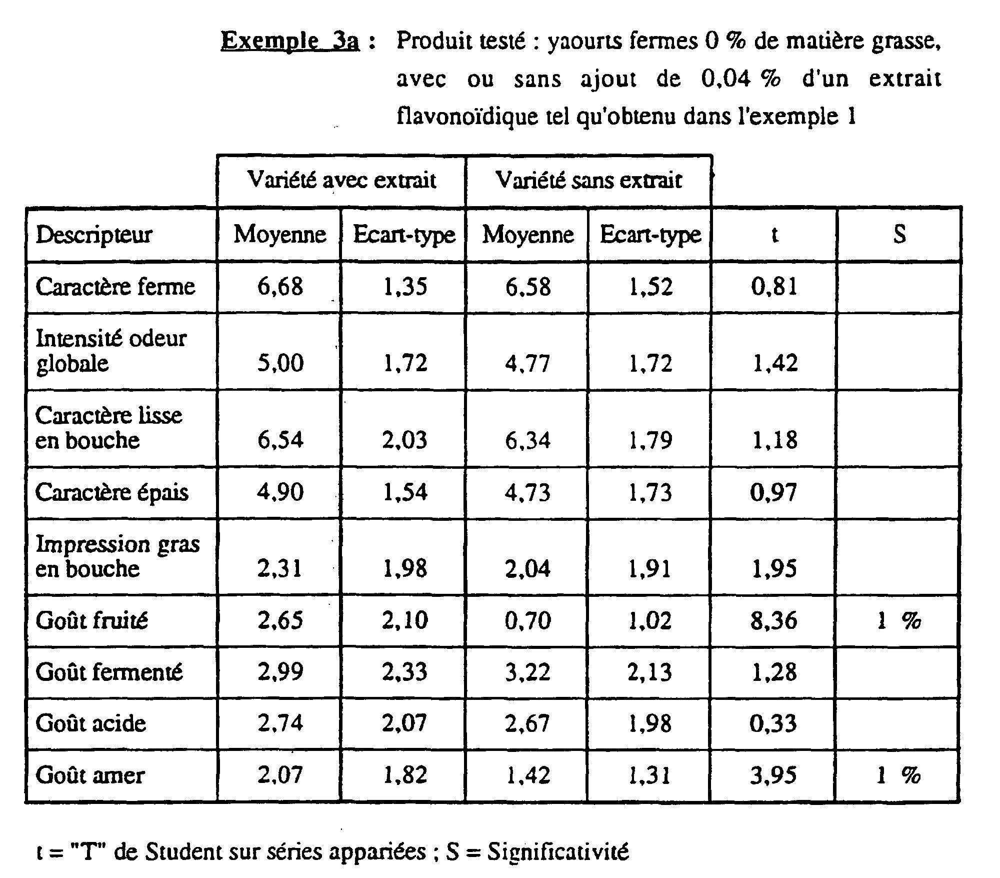

La variété avec l'extrait flavonoïdique se distingue de façon hautement significative comme ayant un goût fruité plus prononcé et un goût amer plus intense que la variété sans extrait flavonoïdique.

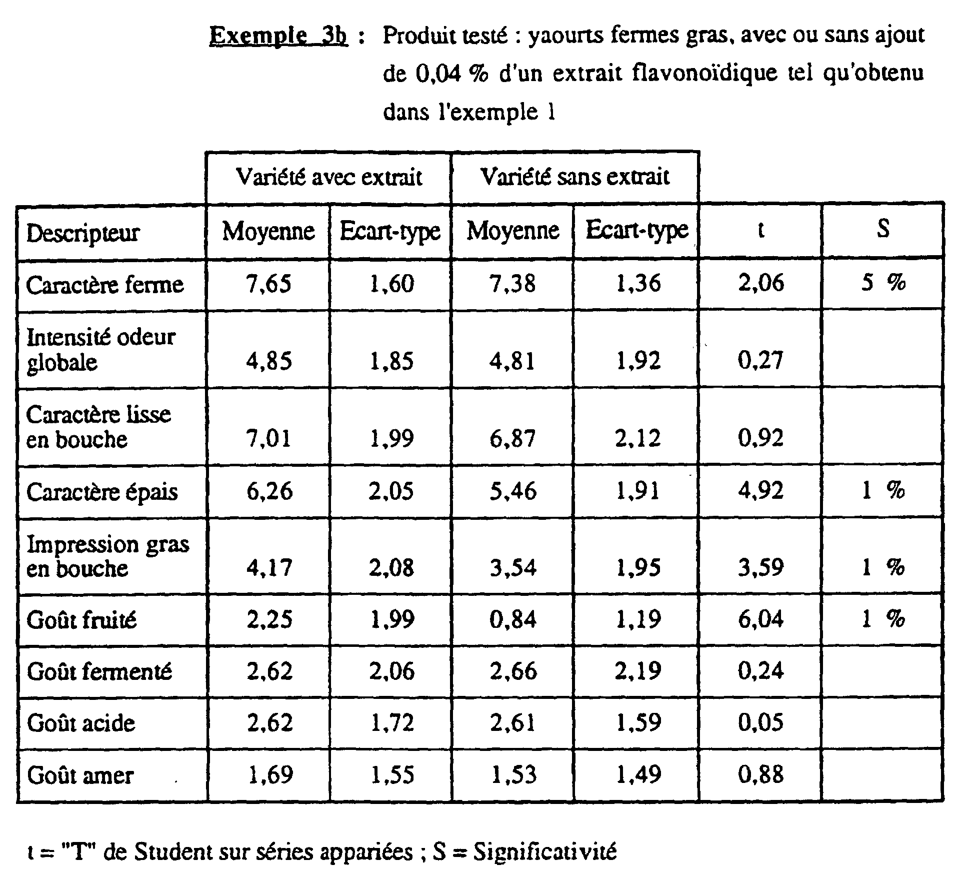

La variété avec l'extrait flavonoïdique se distingue de façon hautement significative comme ayant un caractère plus épais en bouche, une impression de gras en bouche plus intense et un goût fruité plus prononcé et, de façon significative, comme ayant un caractère plus ferme à la cuillère que la variété sans extrait flavonoïdique.

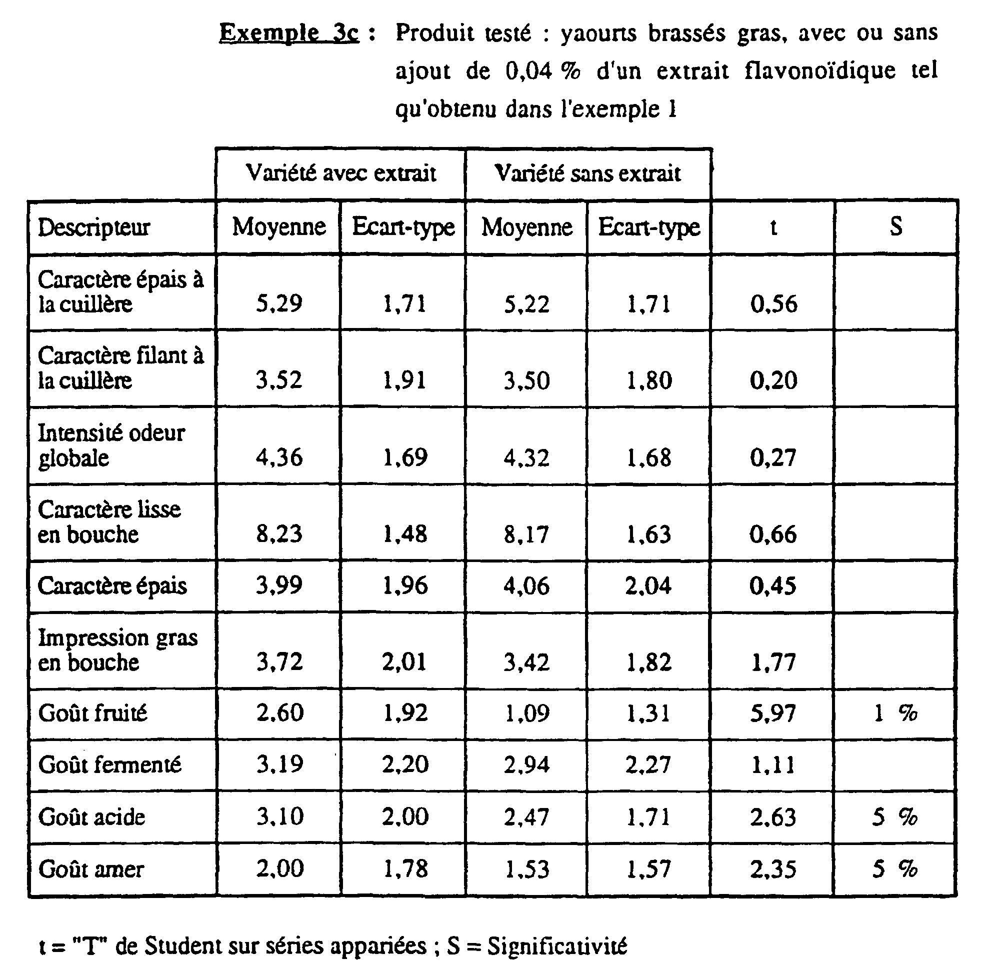

La variété avec l'extrait flavonoïdique se distingue de façon hautement significative comme ayant un goût fruité plus prononcé et, de façon significative, comme ayant un goût plus acide et un goût plus amer que la variété sans extrait flavonoïdique.

## Revendications

1. Extrait flavonoïdique de feuilles de ginkgo biloba, substantiellement dépourvu de terpènes et contenant de 28 à 35 % d'hétérosides flavonoïdiques.

2. Extrait tel que défini à la revendication 1, comprenant au maximum 1 % de terpènes, de préférence au maximum 0,5 % de terpènes, et de 28 à 32 % d'hétérosides flavonoïdiques.

3. Composition aromatisante comprenant, comme ingrédient aromatisant, un extrait selon l'une des revendications 1 à 2, seul ou en association avec d'autres arômes.

4. Produit aromatisé avec une composition selon la revendication 3.

5. Produit selon la revendication 4, qui est un produit laitier et notamment un yaourt.

6. Produit selon la revendication 4, qui est une boisson et notamment une boisson rafraîchissante sans alcool.

7. Produit selon la revendication 4, qui est un produit sucré tel que chewing-gums, bonbons.

8. Utilisation d'une composition selon la revendication 3, dans les produits alimentaires.

9. Utilisation selon la revendication 8, **caractérisée en ce que** le produit alimentaire est un produit laitier et notamment un yaourt.

10. Utilisation selon la revendication 8, pour les boissons telles que boissons rafraîchissantes et nutritives.

11. Utilisation d'un extrait selon l'une des revendications 1 à 2, en tant qu'arôme alimentaire.

## Claims

1. A flavonoid extract from leaves of ginkgo biloba, essentially free of terpenes and comprising from 28 to 35 % flavonoid heterosides.

2. An extract as defined in claim 1, comprising maximally 1 % terpenes, preferably maximally 0.5 % terpenes, and from 28 to 32 % flavonoid heterosides.

3. A flavoring composition comprising as flavoring ingredient an extract according to any of claim 1 to 2, alone or in association with other flavors.

4. A product flavored with a composition according to claim 3.

5. A product according to claim 4 which is a dairy product and especially yogurt.

6. A product according to claim 4 which is a drink and especially a non-alcoholic soft drink.

7. A product according to claim 4 which is a sweetened product such as chewing gum or candy.

8. Use of a composition according to claim 3 in food products.

9. Use of a composition according to claim 8, **characterized in that** the food product is a dairy product and especially yogurt.

10. Use of a composition according to claim 8 for drinks such as refreshing and nutritious drinks.

11. Use of an extract according to any of claims 1 to 2 as food flavoring.

## Patentansprüche

1. Flavonoidextrakt aus Blättern von Ginkgo biloba, der im wesentlichen frei von Terpenen ist und 28 bis 35 % Flavonoidheteroside enthält.

2. Extrakt nach Anspruch 1, der maximal 1 % Terpene, vorzugsweise maximal 0,5 % Terpene, und 28 bis 32 % Flavonoidheteroside enthält.

3. Aromatisierende Zusammensetzung, die als aromatisierenden Bestandteil einen Extrakt nach einem der Ansprüche 1 bis 2 allein oder in Kombination mit anderen Aromen enthält.

4. Mit einer Zusammensetzung nach Anspruch 3 aromatisiertes Produkt.

5. Produkt nach Anspruch 4, das ein Milchprodukt, insbesondere Joghurt, ist.

6. Produkt nach Anspruch 4, das ein Getränk, insbesondere ein alkoholfreies Erfrischungsgetränk, ist.

7. Produkt nach Anspruch 4, das ein gezuckertes Produkt, wie z.B. Kaugummi, Bonbons, ist.

8. Verwendung einer Zusammensetzung nach Anspruch 3, in Nahrungsmitteln.

9. Verwendung nach Anspruch 8, **dadurch gekennzeichnet, daß** das Nahrungsmittel ein Milchprodukt, insbesondere Joghurt, ist.

10. Verwendung nach Anspruch 8 für Getränke, wie z.B. Erfrischungs- oder Nährgetränke.

11. Verwendung eines Extrakts nach einem der Ansprüche 1 bis 2 als Nahrungsmittelaroma.
